# EUROPEAN PATENT APPLICATION

(11) **EP 4 427 757 A1**
(43) Date of publication of application: **11.09.2024**
(21) Application number: 22896101.7
(22) Date of filing: 17.11.2022
(51) Int. Cl.: A61K 35/744, A61K 45/06, A61P 35/00, A23L 33/135, A23K 10/18

(54) **COMPOSITION FOR PREVENTING OR TREATING CANCER OR INFLAMMATION COMPRISING NOVEL LEUCONOSTOC MESENTEROIDES STRAIN, AND METHOD FOR PREVENTING OR TREATING CANCER OR INFLAMMATION USING SAME**

(30) Priority: 17.11.2021 KR 20210158921
(71) Applicant: CJ Bioscience, Inc., Jung-gu Seoul 04527 (KR)
(72) Inventor: MIN, A Rim, Seoul 04560 (KR); KWON, Bo Eun, Seoul 04560 (KR); LEE, Ji Young, Seoul 04560 (KR); KIM, Hyun Jeong, Seoul 04560 (KR); PARK, Hyun Kyung, Seoul 04560 (KR); LEE, Seungwon, Seoul 04560 (KR)
(74) Representative: Michalski Hüttermann & Partner Patentanwälte mbB
(86) International application number: PCT/KR2022/018223
(87) International publication number: WO 2023/090903

(57) **Abstract**

The present disclosure relates to compositions containing a novel *Leuconostoc mesenteroides* strain, a culture thereof, or a component derived therefrom for preventing, alleviating, or treating cancer or inflammation.

## Description

### [Technical Field]

The present disclosure relates to compositions containing a novel *Leuconostoc mesenteroides* strain, a culture thereof, or a component derived therefrom for preventing, alleviating, or treating cancer or inflammation, compositions containing the same for immune enhancement, and methods for preventing or treating cancer or inflammation using the compositions.

### [Background Art]

Cancer, which is an abnormal growth of cells caused by various changes in gene expression, invades and destroys adjacent tissues and metastasizes to distant sites, eventually leading to death.

Cancer has the highest mortality rate worldwide and is the most common cause of death after cardiovascular disease in Western societies. In particular, colorectal cancer, lung cancer, breast cancer, and others show a continuously increasing trend due to the common intake of high-fat diets resulting from the westernization of dietary habits, a rapid increase in environmental pollutants, an increase in alcohol consumption, and the like.

Lactic acid bacteria are widely distributed in the mouths, intestines, and vaginas of humans and animals, feces, and fermented foods, such as yogurt, fast-fermented bean paste, and *kimchi*, and are closely associated with human and animal health. Lactic acid bacteria have been reported to show various health promotion effects, such as intestinal regulation, harmful bacteria inhibition, immunoregulation, blood cholesterol lowering, anti-cancer action, and the like (WO2022-103138 A1).

### [Disclosure]

### [Technical Problem]

The present disclosure relates to compositions containing a novel *Leuconostoc mesenteroides* strain, a culture thereof, or a component derived therefrom for preventing, alleviating, or treating cancer or inflammation, compositions containing the same for immune enhancement, and methods for preventing or treating cancer or inflammation using the compositions.

### [Technical Solution]

An aspect of the present disclosure is to provide a pharmaceutical composition for preventing or treating lung cancer or breast cancer, the composition containing, as an active ingredient, a *Leuconostoc mesenteroides* strain, a culture thereof, or a component derived therefrom.

Another aspect of the present disclosure is to provide a pharmaceutical composition for preventing or treating cancer, the composition containing, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

Still another aspect of the present disclosure is to provide a method for preventing or treating cancer, the method including administering to a subject a pharmaceutical composition of the present disclosure.

Still another aspect of the present disclosure is to provide a pharmaceutical composition for preventing or treating colorectal cancer, the composition containing, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom, wherein the composition is administered in combination with an anticancer agent.

Still another aspect of the present disclosure is to provide a method for preventing or treating lung cancer, breast cancer, or colorectal cancer, the method including administering to a subject a pharmaceutical composition of the present disclosure.

Still another aspect of the present disclosure is to provide a food composition for preventing or alleviating lung cancer, breast cancer, or colorectal cancer, the composition containing, as an active ingredient, a *Leuconostoc mesenteroides* strain, a culture thereof, or a component derived therefrom.

Still another aspect of the present disclosure is to provide a health functional food for preventing or alleviating lung cancer, breast cancer, or colorectal cancer, the health functional food containing, as an active ingredient, a *Leuconostoc mesenteroides* strain, a culture thereof, or a component derived therefrom.

Still another aspect of the present disclosure is to provide a feed composition for preventing or alleviating lung cancer, breast cancer, or colorectal cancer, the composition containing, as an active ingredient, a *Leuconostoc mesenteroides* strain, a culture thereof, or a component derived therefrom.

Still another aspect of the present disclosure is to provide a food composition for preventing or alleviating cancer or inflammation, the composition containing, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

Still another aspect of the present disclosure is to provide a health functional food for preventing or alleviating cancer or inflammation, the health functional food containing, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

Still another aspect of the present disclosure is to provide a feed composition for preventing or alleviating cancer or inflammation, the composition containing, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

Still another aspect of the present disclosure is to provide a pharmaceutical composition for immune enhancement, the composition containing, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

Still another aspect of the present disclosure is to provide a food composition for immune enhancement, the composition containing, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

Still another aspect of the present disclosure is to provide a health functional food for immune enhancement, the health functional food containing, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

Still another aspect of the present disclosure is to provide a feed composition for immune enhancement, the composition containing, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

### [Advantageous Effects]

The novel *Leuconostoc mesenteroides* strains according to the present disclosure exhibit anticancer and anti-inflammatory effects, and exhibit an immune enhancing effect, such as increasing the secretion of the immune enhancing cytokines IL-6, IL-12p70, and IFNγ through the enhancement of CD8 T cell potency, and thus can be applied for prevention or treatment of cancer or inflammation or for immune enhancement.

### [Brief Description of Drawings]

FIG. 1 illustrates enzymatic activity of *Leuconostoc mesenteroides* CJRS-10671 and CJRS-10672 strains.
FIG. 2 illustrates hemolytic activity of CJRS-10671 and CJRS-10672 strains.
FIG. 3 illustrates intestinal adhesion of CJRS-10671 and CJRS-10672 strains.
FIG. 4 illustrates interleukin (IL)-6 secretion by CJRS-10671 and CJRS-10672 strains in macrophage lines.
FIG. 5 illustrates IL-12p70 secretion by CJRS-10671 and CJRS-10672 strains in human peripheral blood mononuclear cells (PBMCs).
FIG. 6 illustrates interferon-gamma (IFNγ) secretory capacity of CD8 T cells by CJRS-10671 and CJRS-10672 strains in mouse splenocytes and human PBMCs.
FIG. 7 illustrates the cell cycle arrest effect of cancer cells by CJRS-10671 and CJRS-10672 strains.
FIG. 8 illustrates the expression of cyclin B1 and cyclin dependent kinase 1 (CDK1), which are cell cycle arrest-related factors in cancer cells, by CJRS-10671 and CJRS-10672 strains.
FIG. 9 illustrates cancer cell death by CJRS-10671 and CJRS-10672 strains.
FIG. 10 illustrates a tumor inhibitory effect by the administration of CJRS-10671 and CJRS-10672 in a mouse lung cancer model (LLC1).
FIG. 11 illustrates IFNγ secretory capacity of CD8 T cells by the administration of CJRS-10671 in a mouse lung cancer model (LLC1).
FIG. 12 illustrates IFNγ secretory capacity of CD8 T cells by the administration of CJRS-10672 in a mouse lung cancer model (LLC1).
FIG. 13 illustrates IFNγ secretory capacity of CD8 T cells in tumor tissue by the administration of CJRS-10671 and CJRS-10672 in a mouse lung cancer model (LLC1).
FIG. 14 illustrates changes of myeloid-derived suppressor cells (MDSCs) as immune suppressor cells by the administration of CJRS-10671 and CJRS-10672 in a mouse lung cancer model (LLC1).
FIG. 15 illustrates immune cell change profiling by the administration of CJRS-10671 in a mouse lung cancer model (LLC1).
FIG. 16 illustrates immune cell change profiling by the administration of CJRS-10672 in a mouse lung cancer model (LLC1).
FIG. 17 illustrates a tumor inhibitory effect by the administration of CJRS-10672 in a mouse breast cancer model (4T1).
FIG. 18 illustrates a change of MDSCs as immune suppressor cells by the administration of CJRS-10672 in a mouse breast cancer model (4T1).

### [Best Mode for Carrying Out the Invention]

The present disclosure will be specifically described as follows. Each description and embodiment in the present disclosure may also be applied to other descriptions and embodiments. That is, all combinations of various elements in the present disclosure fall within the scope of the present disclosure. Furthermore, the scope of the present disclosure is not limited by the specific description below. Furthermore, those skilled in the art will recognize, or be able to ascertain, using no more than routine experimentation, many equivalents to the specific embodiments of the disclosure described herein. Furthermore, these equivalents should be intended to fall within the present disclosure.

In accordance with an aspect of the present disclosure, there is provided a pharmaceutical composition for preventing or treating lung cancer or breast cancer, the composition containing, as an active ingredient, a *Leuconostoc mesenteroides* strain, a culture thereof, or a component derived therefrom.

As used herein, the term "*Leuconostoc mesenteroides*" refers to a lactic acid bacterium with a round spherical shape and is known as probiotics or a lactic acid bacterium that functions to ferment a food such as *kimchi.*

The *Leuconostoc mesenteroides* strain of the present disclosure may be at least one selected from CJRS-10671 deposited under accession number KCCM13059P and CJRS-10672 deposited under accession number KCCM13060P.

The CJRS-10671 strain of the present disclosure may have high enzymatic activity of β-galactosidase, α-glucosidase, and β-glucosidase, and the CJRS-10672 strain of the present disclosure may have enzymatic activity of β-glucosidase.

In addition, the strain of the present disclosure may have an intestine regulating effect and an immune enhancing effect.

The strain of the present disclosure contained in the composition according to the present disclosure may be present as live cells or dead cells and may be present in a dried or freeze-dried form. The culture of the present disclosure may include a live cell culture (that is, a product obtained by culturing live cells in a medium), a culture obtained by killing live cells in the live cell culture, or a culture filtrate (that is, a product obtained by removing dead cells or live cells from the culture). The component derived from the strain of the present disclosure may include a cytoplasmic fraction obtained by cell disruption and containing extracellular vesicles (EVs) or the like. Additionally, forms of lactic acid bacteria suitable to be contained in the pharmaceutical composition, formulating methods therefor, isolation methods of strain-derived components, and the like are well known to those skilled in the art.

Specifically, the strain of the present disclosure may be a strain obtained by inoculation to 0.1% to 10% in an MRS liquid medium and culture at 25°C to 40°C for 4 to 72 hours.

The composition of the present disclosure may be administered in combination with an anticancer agent.

In accordance with another aspect of the present disclosure, there is provided a pharmaceutical composition for preventing or treating colorectal cancer, the composition containing, as an active ingredient, *Leuconostoc mesenteroides* CJRS-10671 (accession number: KCCM13059P) or *Leuconostoc mesenteroides* CJRS-10672 (accession number: KCCM13060P);, a culture thereof; or a component derived therefrom, wherein the composition is administered in combination with an anticancer agent.

The strain, culture, and derived ingredients are as described above.

The strain of the present disclosure can exhibit anticancer, anti-inflammatory, and immune enhancing effects.

As used herein, the term "preventing" refers to all actions that inhibit or delay the development of cancer by the administration of the composition of the present disclosure, and the term "treating" refers to all actions that improve or beneficially change symptoms of cancer by the administration of the composition of the present disclosure.

In the present disclosure, the cancer may include any type of cancer that is known in the art without limitation, and examples thereof may include lung cancer (e.g., non-small cell lung cancer, small cell lung cancer, malignant mesothelioma), mesothelioma, pancreatic cancer (e.g., pancreatic duct cancer, pancreatic endocrine tumor), pharyngeal cancer, laryngeal cancer, esophagus cancer, gastric cancer (e.g., papillary adenocarcinoma, mucinous adenocarcinoma, adenosquamous carcinoma), duodenal cancer, small intestinal cancer, colorectal cancer (e.g., colon cancer, rectal cancer, anal cancer, familial colorectal cancer, hereditary nonpolyposis colorectal cancer, gastrointestinal stromal tumor), breast cancer (e.g., invasive ductal carcinoma, non-invasive ductal carcinoma, inflammatory breast cancer), ovarian cancer (e.g., ovarian epithelial carcinoma, extragonadal germ cell tumor, ovarian germ cell tumor, ovarian low malignant potential tumor), testis tumor, prostate cancer (e.g., hormone-dependent prostate cancer, non-hormone dependent prostate cancer), liver cancer (e.g., hepatocyte cancer, primary liver cancer, extrahepatic bile duct cancer), thyroid cancer (e.g., medullary thyroid carcinoma), kidney cancer (e.g., renal cell carcinoma, transitional cell carcinoma of renal pelvis and ureter), uterine cancer (e.g., uterine cervical cancer, cancer of uterine body, uterus sarcoma), brain tumor (e.g., medulloblastoma, glioma, pineal astrocytoma, pilocytic astrocytoma, diffuse astrocytoma, anaplastic astrocytoma, hypophysial adenoma), retina blastoma, skin cancer (e.g., basalioma, malignant melanoma), sarcoma (e.g., rhabdomyosarcoma, leiomyosarcoma, soft tissue sarcoma), malignant bone tumor, urinary bladder cancer, hematologic cancer (e.g., multiple myeloma, leukemia, malignant lymphoma, Hodgkin's disease, chronic bone marrow proliferative disease), cancer of unknown primary, and the like, but are not limited thereto, and specifically, may be lung cancer, breast cancer, or colorectal cancer.

The pharmaceutical composition of the present disclosure may be administered via any general route as long as the composition can reach a target tissue. Various manners of administration may be considered including intraperitoneal administration, intravenous administration, intramuscular administration, subcutaneous administration, intradermal administration, oral administration, topical administration, intranasal administration, intrapulmonary administration, and intrarectal administration, but the present disclosure is not limited to such exemplary manners of administration. Preferably, the composition may be administered in the form of an injection. In addition, the pharmaceutical composition may be administered by a specific device that enables an active ingredient to move to a target tissue.

The pharmaceutical composition of the present disclosure may further contain a pharmaceutically acceptable salt. For oral administration, examples of the pharmaceutically acceptable carrier may include a binder, a lubricant, a disintegrant, an excipient, a solubilizer, a dispersant, a stabilizer, a suspending agent, a coloring agent, and a flavoring agent. For an injection, examples of the pharmaceutically acceptable carrier may include a buffer, a preservative, an analgesic, a solubilizer, an isotonic agent, and a stabilizer. For topical administration, examples of the pharmaceutically acceptable carrier may include a base, an excipient, a lubricant, and a preservative. The pharmaceutical composition of the present disclosure may be prepared as a variety of formulations in combination with the above-described pharmaceutically acceptable carriers. For example, for oral administration, the pharmaceutical composition may be formulated into tablets, troches, capsules, elixirs, suspensions, syrups, or wafers. For an injection, the pharmaceutical composition may be formulated into an ampule as a single-dose formulation or a multiple-dose formulation such as a multi-dose container. The composition may be also formulated into solutions, suspensions, tablets, pills, capsules, and sustained release preparations.

Meanwhile, examples of the carrier, excipient, and diluent suitable for formulation include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oils. In addition, the composition may further contain a filler, an anticoagulant, a lubricant, a humectant, a flavoring agent, and an antiseptic.

The pharmaceutical composition of the present disclosure may be administered at a pharmaceutically effective amount.

As used herein, the term "pharmaceutically effective amount" refers to an amount that is sufficient to treat a disease at a reasonable benefit/risk ratio applicable to medical treatment, and the level of the effective dose may be determined depending on factors including the kind, disease severity, age and sex of an individual, the type of disease, the activity of a drug, the sensitivity to a drug, the time of administration, the route of administration, the rate of excretion, the duration of treatment, and drugs used in combination with the composition, and other factors well known in the medical field. The pharmaceutical composition of the present disclosure may be administered as an individual medicine or in combination with another medicine and may be administered sequentially or simultaneously with conventional medicines. In addition, the composition may be administered once or multiple times. It is important to administer an amount capable of obtaining a maximum effect with a minimal amount without side effects in consideration of all of the factors, and the amount thereof may easily be determined by those skilled in the art. A preferable dose of the pharmaceutical composition of the present disclosure may be determined by several related factors including the type of cancer to be treated, the route of administration, patient's age, sex, weight, and disease severity as well as by the type of anti-cancer agent. When the pharmaceutical composition of the present disclosure is administered in combination with an anticancer agent, the dose of the anticancer agent administered in combination can be reduced, thereby reducing side effects of the anticancer agent and increasing the therapeutic compliance of a patient. The administration may be carried out once or several times in divided doses per day.

The pharmaceutical composition of the present disclosure may be used in the form of an animal medicine as well as a pharmaceutical product applied to humans. Herein, the animal includes livestock and pets.

In the present disclosure, the pharmaceutical composition of the present disclosure may be administered in combination with an anticancer agent. Examples of the anticancer agent may include taxane-based anticancer agents, statins, alkylating agents, platinum-based drugs, antimetabolites, antibiotics, vinca alkaloid-based anticancer agents, targeted therapeutics, anticancer immunotherapeutic agents, cancer vaccines, cell therapeutics, oncolytic viruses, and combinations thereof, but are not limited thereto, and specifically, the anticancer agent may be an anticancer immunotherapeutic agent.

Examples of the taxane-based anticancer agents may be paclitaxel, docetaxel, larotaxel, cabazitaxel, and the like, but are not limited thereto.

Examples of the statins may be simvastatin, atorvastatin, lovastatin, fluvastatin, cerivastatin, pitavastatin, and the like, but are not limited thereto.

Examples of the alkylating agents may be nitrogen mustard-based drugs, ethylenimine- and methylmelamine-based drugs, methyl hydrazine derivatives, alkyl sulfonate-based drugs, nitrosourea-based drugs, triazine-based drugs, and the like, but are not limited thereto.

Examples of the platinum-based drugs may be cisplatin, carboplatin, oxaliplatin, and the like, but are not limited thereto.

Examples of the antimetabolites may be folate antagonist-based drugs, purine antagonist-based drugs, pyrimidine antagonist-based drugs, and the like, but are not limited thereto.

Examples of the antibiotics may be etoposide, topotecan, irinotecan, idarubicin, epirubicin, dactinomycin, doxorubicin, Adriamycin, daunorubicin, bleomycin, mitomycin C, mitoxantrone, and the like, but are not limited thereto.

Examples of the vinca alkaloid-based anticancer drugs may be vincristine, vinblastine, vinorelbine, and the like, but are not limited thereto.

Examples of the targeted therapeutics may be epidermal growth factor receptor (EGFR) targeted therapeutics, human epidermal growth factor receptor 2 (HER2) targeted therapeutics, CD20 (B cell marker) targeted therapeutics, CD33 (myeloid cell surface antigen) targeted therapeutics, CD52 (cluster of differentiation 52) targeted therapeutics, CD30 (tumor necrosis factor receptor superfamily member 8) targeted therapeutics, breakpoint cluster region protein-tyrosine-protein kinase (bcr-abl)/tyrosine kinase receptor (c-Kit) targeted therapeutics, anaplastic lymphoma receptor tyrosine kinase (ALK) targeted therapeutics, antiangiogenics targeted therapeutics, mammalian target of rapamycin (mTOR) targeted therapeutics, cyclin-dependent kinase 4/6 (CDK4/6) targeted therapeutics, poly (ADP-ribose) polymerase (PARP) targeted therapeutics, proteasome inhibitors, tyrosine kinase) antagonists, protein kinase C inhibitors, farnesyl transferase inhibitors, and the like, but are not limited thereto.

Examples of the anticancer immunotherapeutic agents may be anti-programmed cell death protein 1 (anti-PD-1), anti-programmed cell death-ligand 1 (anti-PD-L1), cytotoxic T lymphocyte associated antigen 4 (CTLA4), CTLA4/B7-1/B7-2 interaction inhibitors, cluster of differentiation 47/signal-regulatory protein (CD47/SIRP) interaction inhibitors, anti-T-cell immunoglobulin and mucin domain 3 (anti-Tim3), anti-lymphocyte activating gene 3 (anti-LAG3), and the like, but are not limited thereto.

Specifically, the anticancer agent of the present disclosure may be, but is not limited to, an anti-PD-1 immunotherapeutic agent.

In the present disclosure, the co-administration may be administering the pharmaceutical composition and an anticancer agent simultaneously, sequentially, or reverse-sequentially, but the order of administration is not limited and falls within the scope of the present disclosure as long as the two are administered at dosing intervals capable of exhibiting pharmacological effects of the two. The pharmaceutical composition and an anticancer agent may be separately prepared into formulations or may be prepared into one formation, but are not limited thereto.

In the present disclosure, the administration of the pharmaceutical composition of the present disclosure may be performed in combination with anticancer therapy. Examples of the anticancer therapy may be radiotherapy, neoadjuvant therapy, chemotherapy, targeted therapy, photodynamic therapy, and the like, but are not limited thereto, and may include all types of anticancer therapy known in the art.

In the present disclosure, the pharmaceutical composition of the present disclosure may exhibit an anticancer effect. The anticancer effect may include a cancer cell killing or tumor growth inhibitory effect (Examples 9 and 10-1).

For example, the pharmaceutical composition of the present disclosure can inhibit the cell cycle of cancer cells to show a cancer cell killing or tumor growth inhibitory effect, leading to an anticancer effect (Example 8).

As another example, the pharmaceutical composition of the present disclosure may exhibit an anti-inflammatory or immune enhancing effect by increasing the secretion of interleukin (IL)-6, IL-12p70, and interferon-gamma (IFNγ), which are immune enhancing cytokines (Example 7).

In accordance with still another aspect of the present disclosure, there is provided a method for preventing or treating lung cancer, breast cancer, or colorectal cancer, the method including administering to a subject the pharmaceutical composition of the present disclosure.

The pharmaceutical composition and cancer are as described above.

The treatment method of the present disclosure includes administering a pharmaceutically effective amount of the pharmaceutical composition to a subject suspected of having cancer. The subject may mean any mammal including dog, cow, horse, rabbit, mouse, rat, chicken, and human, but the mammal of the present disclosure is not limited to these examples. The pharmaceutical composition may be administered via parenteral, subcutaneous, intraperitoneal, intrapulmonary, and intranasal routes. For topical treatment, the pharmaceutical composition may be administered by an appropriate method including intralesional administration, if necessary. The parenteral injection includes intramuscular, intravenous, intraarterial, intraperitoneal, or subcutaneous administration. A preferable formulation is a formulation of an intravenous injection, subcutaneous injection, intradermal injection, intramuscular injection, or instillation. A preferable dose of the pharmaceutical composition of the present disclosure may vary depending on the condition and weight of the subject, severity of the illness, drug type, and route and period of administration, and may be appropriately determined by those skilled in the art.

In accordance with still another aspect of the present disclosure, there is provided a food composition for preventing or alleviating lung cancer, breast cancer, or colorectal cancer, the composition containing, as an active ingredient, a *Leuconostoc mesenteroides* strain, a culture thereof, or a component derived therefrom.

In accordance with still another aspect of the present disclosure, there is provided a health functional food for preventing or alleviating lung cancer, breast cancer, or colorectal cancer, the health functional food containing, as an active ingredient, a *Leuconostoc mesenteroides* strain, a culture thereof, or a component derived therefrom.

The strain, culture, derived component, and cancer are as described above.

The food composition may be in the form of a health functional food or a food additive.

As used herein, the term "health functional food" refers to a food that is manufactured and processed using raw materials or components having functionality beneficial to the human body (No. 1 of Article 3), and the term "functionality" refers to controlling nutrients for the structure or functions of the human body or providing beneficial effects for health purposes, such as physiological actions (No. 2 of the same article), according to the Health Functional Food Act.

The food composition of the present disclosure may further contain a food additive, and the suitability thereof as a "food additive", unless otherwise specified, is determined according to the standards and criteria for the corresponding item in accordance with the General Rules and General Test Methods of the Food Additive Code approved by the Ministry of Food and Drug Safety.

The items listed on the "Food Additives Code" may include, for example: chemical synthetic products, such as ketones, glycine, potassium citrate, nicotinic acid, and cinnamic acid; natural additives, such as a persimmon pigment, a licorice root extract, crystalline cellulose, and guar gum; and mixed preparations, such as a sodium L-glutamate preparation, an alkali agent for addition to noodles, a preservative preparation, and a tar color preparation.

Examples of the food containing an active ingredient of the present disclosure may include: confectionery, such as bread, rice cakes, dried snacks, candies, chocolates, chewing gum, and jams; ice cream products, such as ice creams, frozen desserts, and ice cream powder; dairy products, such as milk, low-fat milk, lactose degradation milk, processed milk, goat milk, fermented milk, butter milk, concentrated milk, milk creams, natural cheese, processed cheese, powdered milk, and whey; meat products, such as processed meat products, processed egg products, and hamburgers; fish meat products, such as fish cakes and fish meat processed products, for example, ham, sausage, and bacon; noodles, such as ramen, dried noodles, fresh noodles, instant fried noodles, gelatinized dried noodles, modified cooked noodles, frozen noodles, and pastas; beverages, such as fruit beverages, vegetable beverages, carbonated drinks, soybean milk, lactobacillus beverages including yogurts, and mixed beverages; seasoning foods, such as soy sauce, fermented bean paste, red pepper paste, black bean paste, fast-fermented bean paste, mixed paste, vinegar, sauces, tomato ketchup, curries, and dressings; and fermented foods, but are not limited thereto.

In addition to above, the food composition of the present disclosure may contain various nutrients, vitamins, electrolytes, flavoring agents, colorants, pectic acid and salts thereof, alginic acid and salts thereof, organic acids, protective colloid thickeners, pH adjusters, stabilizers, preservatives, glycerin, alcohols, carbonating agents used in carbonated drinks, and the like. In addition, the food composition of the present disclosure may contain flesh for manufacturing natural fruit juices, fruit juice beverages, and vegetable beverages. These ingredients may be used alone or in combination.

In accordance with still another aspect of the present disclosure, there is provided a feed composition for preventing or alleviating lung cancer, breast cancer, or colorectal cancer, the composition containing, as an active ingredient, a *Leuconostoc mesenteroides* strain, a culture thereof, or a component derived therefrom.

The strain, culture, derived component, and cancer are as described above.

The feed composition may be in the form of a feed additive composition in addition to a feed composition.

When the composition is manufactured as a feed additive, the composition may be manufactured as a highly concentrated solution of 20% to 90% or in the form of a powder or granules. The feed additive may further contain any one or one or more of: organic acids, such as citric acid, fumaric acid, adipic acid, lactic acid, and malic acid; phosphate salts, such as sodium phosphate, potassium phosphate, acidic pyrophosphate, and polyphosphates; or natural anti-oxidants, such as polyphenols, catechin, alpha-tocopherol, rosemary extracts, vitamin C, green tea extracts, licorice extracts, chitosan, tannic acid, and phytic acid. When the composition is manufactured as a feed, the composition may be formulated in a common feed form and may contain common feed ingredients together.

The feed and feed additive may further contain: grains, for example, powdered or crushed wheat, oat, barley, corn, and rice; plant protein feeds, for example, feeds having rape, bean and sunflower as main ingredients; animal protein feeds, for example, powdered blood, powdered meat, powered bone, and powdered fish; and sugars and dairy products, for example, dried components including various types of powdered milk and powdered whey, and may further contain, in addition the above, nutritional supplements, digestion and absorption enhancers, growth accelerators, and the like.

The feed additive may be administered, to animals, alone or in combination with other feed additives among edible carriers. The feed additive may easily be administered, to animals, as a top dressing, by directly mixing with an animal feed, or as an oral formulation separately from the feed. When the feed additive is administered separately from the animal feed, the feed additive may be prepared as an immediate release or sustained release formulation, in combination with a pharmaceutically acceptable edible carrier well known in the art. The edible carrier may be a solid or liquid, for example, corn starch, lactose, sucrose, bean flakes, peanut oil, olive oil, sesame oil, and propylene glycol. When a solid carrier is used, the feed additive may be in the form of tablets, capsules, powder, troches, lozenges, or non-dispersed top dressings. When a liquid carrier is used, the feed additive may be in the formulation of gelatin soft capsules, syrups, suspensions, emulsions, or solutions.

The feed and feed additive may contain adjuvants, such as preservatives, stabilizers, humectants, emulsifiers, and solution promoters. The feed additive may be used by addition to an animal feed through soaking, spraying or mixing.

The feeds or feed additives of the present disclosure may be applied to feeds for numerous animals including mammals, poultry, and fish.

The feeds or feed additives of the present disclosure may be used for: mammals, such as pigs, cows, horses, sheep, rabbits, goats, rodents and experimental rodents including rats, hamsters, and guinea pigs, as well as pets (e.g., dogs and cats); poultry, such as chickens, turkeys, ducks, geese, pheasants, and quails; and fish, such as carp, crucian carp, and trout, but are not limited thereto.

In accordance with still another aspect of the present disclosure, there is provided a pharmaceutical composition for preventing or treating cancer, the composition containing, as an active ingredient, *Leuconostoc mesenteroides* CJRS-10671 (accession number: KCCM13059P) or *Leuconostoc mesenteroides* CJRS-10672 (accession number: KCCM13060P), a culture thereof, or a component derived therefrom.

In accordance with still another aspect of the present disclosure, there is provided a method for preventing or treating cancer, the method including administering to a subject the pharmaceutical composition of the present disclosure.

In accordance with still another aspect of the present disclosure, there is provided a food composition for preventing or alleviating cancer or inflammation, the composition containing, as an active ingredient, *Leuconostoc mesenteroides* CJRS-10671 (accession number: KCCM13059P) or *Leuconostoc mesenteroides* CJRS-10672 (accession number: KCCM13060P), a culture thereof, or a component derived therefrom.

In accordance with still another aspect of the present disclosure, there is provided a health functional food for preventing or alleviating cancer or inflammation, the health functional food containing, as an active ingredient, *Leuconostoc mesenteroides* CJRS-10671 (accession number: KCCM13059P) or *Leuconostoc mesenteroides* CJRS-10672 (accession number: KCCM13060P), a culture thereof, or a component derived therefrom.

In accordance with still another aspect of the present disclosure, there is provided a feed composition for preventing or alleviating cancer or inflammation, the composition containing, as an active ingredient, *Leuconostoc mesenteroides* CJRS-10671 (accession number: KCCM13059P) or *Leuconostoc mesenteroides* CJRS-10672 (accession number: KCCM13060P), a culture thereof, or a component derived therefrom.

In accordance with still another aspect of the present disclosure, there is provided a pharmaceutical composition for immune enhancement, the composition containing, as an active ingredient, *Leuconostoc mesenteroides* CJRS-10671 (accession number: KCCM13059P) or *Leuconostoc mesenteroides* CJRS-10672 (accession number: KCCM13060P), a culture thereof, or a component derived therefrom.

In accordance with still another aspect of the present disclosure, there is provided a food composition for immune enhancement, the composition containing, as an active ingredient, *Leuconostoc mesenteroides* CJRS-10671 (accession number: KCCM13059P) or *Leuconostoc mesenteroides* CJRS-10672 (accession number: KCCM13060P), a culture thereof, or a component derived therefrom.

In accordance with still another aspect of the present disclosure, there is provided a health functional food for immune enhancement, the health functional food containing, as an active ingredient, *Leuconostoc mesenteroides* CJRS-10671 (accession number: KCCM13059P) or *Leuconostoc mesenteroides* CJRS-10672 (accession number: KCCM13060P), a culture thereof, or a component derived therefrom.

In accordance with still another aspect of the present disclosure, there is provided a feed composition for immune enhancement, the composition containing, as an active ingredient, *Leuconostoc mesenteroides* CJRS-10671 (accession number: KCCM13059P) or *Leuconostoc mesenteroides* CJRS-10672 (accession number: KCCM13060P), a culture thereof, or a component derived therefrom.

In the composition of the present disclosure, the strain, culture, derived component, cancer, inflammation, immune enhancement, and the like are as described above.

### [Detailed Description of the Invention]

Hereinafter, the present disclosure will be described in detail with reference to examples. However, these examples are merely preferable embodiments for illustrating the present disclosure, and therefore are not intended to limit the scope of right of the present disclosure. Meanwhile, the technical matters not described herein can be sufficiently understood and easily implemented by those skilled in the art or similar technical fields of the present disclosure. Furthermore, throughout the entire specification, many papers and patent documents are referenced and their citations are provided. The disclosures of cited papers and patent documents are incorporated in their entirety by reference into the present specification, and the level of the technical field within which the present disclosure falls and details of the present disclosure are explained more clearly.

### Example 1: Sugar fermentation characteristics of Leuconostoc mesenteroides strain

The *Leuconostoc mesenteroides* CJRS-10671 and CJRS-10672 strains isolated from *kimchi* were cultured using MRS liquid medium and Agar medium from Difco. Each medium was prepared according to a protocol and then sterilized at 121 °C for 15 minutes. Two days before the experiment, a stock of each strain was inoculated at 1% in MRS liquid medium and statically cultured at 37°C for 20 hours, and then inoculated again at 2% in MRS liquid medium (activation) and cultured at 37°C for 20 hours.

The CJRS-10671 and CJRS-10672 strains thus cultured were examined for sugar fermentation characteristics for 49 kinds of carbohydrates by using an API 50 CHL kit (Biomerieux, France). Specifically, the strains subcultured for two passages were washed with phosphate buffered saline (PBS), and then each strain was suspended in API 50 CHL medium to McFarland standard 2.0, inoculated onto strips, and then cultured at 37°C for 24 hours. A yellow color due to the use of carbohydrates was read as indicating a positive reaction, and a purple color due to the non-use of carbohydrates was read as indicating a negative reaction.

**TABLE 1**

| No. | Type of test | CJRS-10671 | CJRS-10672 | No. | Type of test | CJRS-10671 | CJRS-10672 |
|---|---|---|---|---|---|---|---|
| 0 | Control | - | - | 25 | Esculin | ++ | ++ |
| 1 | Glycerol | - | - | 26 | Salicin | ++ | - |
| 2 | Erythritol | - | - | 27 | D-Cellibiose | ++ | - |
| 3 | D-Arabinose | - | - | 28 | D-Maltose | ++ | ++ |
| 4 | L-Arabinose | ++ | ++ | 29 | D-Lactose | - | - |
| 5 | D-Ribose | ++ | ++ | 30 | D-Melibiose | ++ | ++ |
| 6 | D-Xylose | ++ | ++ | 31 | D-Saccharose (sucrose) | ++ | ++ |
| 7 | L-Xylose | - | - | 32 | D-Trehalose | ++ | ++ |
| 8 | D-Adonitol | - | - | 33 | Inullin | - | - |
| 9 | Methyl-β-D-xylopyranoside | - | - | 34 | D-Melezitose | - | - |
| 10 | D-Galactose | - | - | 35 | D-Raffinose | ++ | ++ |
| 11 | D-Glucose | ++ | ++ | 36 | Amidon | - | - |
| 12 | D-Fructose | ++ | ++ | 37 | Glycogen | - | - |
| 13 | D-Mannose | ++ | ++ | 38 | Xylitol | - | - |
| 14 | L-Sorbose | - | - | 39 | Gentibiose | ++ | - |
| 15 | L-Rhamnose | - | - | 40 | D-Turanose | ++ | - |
| 16 | Dulcitol | - | - | 41 | D-Lyxose | - | - |
| 17 | Inositol | - | - | 42 | D-Tagatose | - | - |
| 18 | D-Mannitol | + | + | 43 | D-Fucose | - | - |
| 19 | D-Sorbitol | + | - | 44 | L-Fucose | - | - |
| 20 | Methyl-α-D-mannopyranoside | - | - | 45 | D-Arabitol | - | - |
| 21 | Methyl-α-D-glucopyranoside | ++ | ++ | 46 | L-Arabitol | - | - |
| 22 | *N*-Acetyl glucosamine | ++ | ++ | 47 | Potassium gluconate | + | - |
| 23 | Amygdalin | + | - | 48 | Potassium 2-ketogluconate | - | + |
| 24 | Arbutine | + | + | 49 | Potassium 5-ketoglyconate | - | - |
| (++): positive reaction (yellow), No. 25 (black), (+): weak reaction, (-): negative reaction (purple) | | | | | | | |

### Example 2: Enzymatic activity of strains

To analyze biochemical characteristics of the CJRS-10671 and CJRS-10672 strains of Example 1, the strains were examined for 19 types of enzymatic activity by using an API ZYM kit (Biomerieux, France).

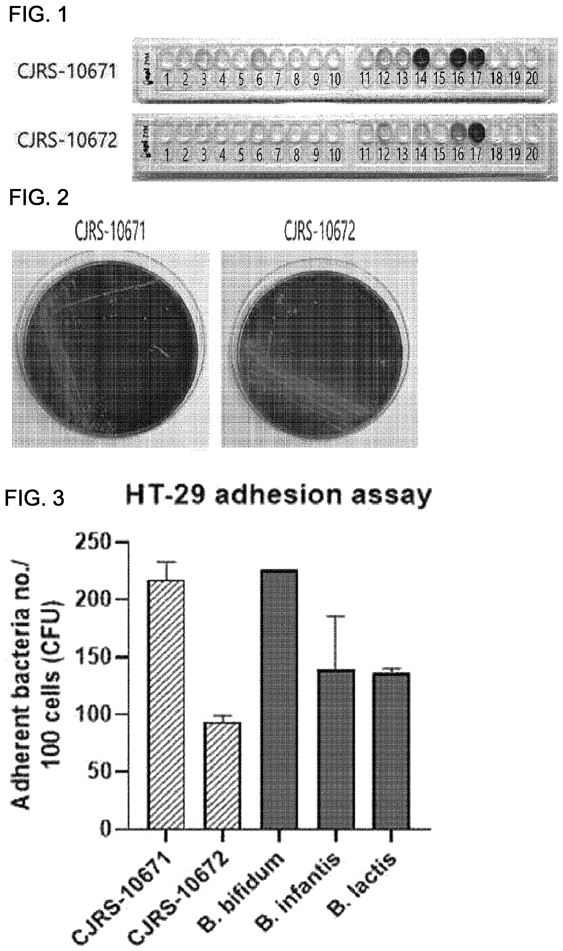

As a result, as shown in Table 2 below and FIG. 1, it was confirmed that CJRS-10671 showed high enzymatic activity in β-galactosidase, α-glucosidase, and β-glucosidase, and CJRS-10672 showed high enzymatic activity in β-glucosidase.

**TABLE 2**

| No. | Enzyme | CJRS-10671 | CJRS-10672 |
|---|---|---|---|
| 1 | Control | | |
| 2 | Alkaline phosphatase | 0 | 0 |
| 3 | Esterase (c4) | 1 | 1 |
| 4 | Esterase lipase (C8) | 0 | 0 |
| 5 | Lipase (c14) | 0 | 0 |
| 6 | Leucine arylamidase | 1 | 1 |
| 7 | Valine arylamidase | 0 | 0 |
| 8 | Cystine arylamidase | 0 | 0 |
| 9 | Trypsin | 0 | 0 |
| 10 | α-Chymotrypsin | 0 | 0 |
| 11 | Acid phosphatase | 0.5 | 0.5 |
| 12 | Naphthol-AS-BI-phosphohydrolase | 1 | 1 |
| 13 | α-Galactosidase | 0.5 | 0 |
| 14 | β-Galactosidase | 4 | 1 |
| 15 | β-Glucuronidase | 0 | 0 |
| 16 | α-Glucosidase | 5 | 2 |
| 17 | β-Glucosidase | 5 | 5 |
| 18 | *N*-Acetyl-β-glucosaminidase | 0 | 0 |
| 19 | α-Mannosidase | 0 | 0 |
| 20 | α-Fucosidase | 0 | 0 |

### Example 3: Hemolytic activity of strains

To determine whether the CJRS-10671 and CJRS-10672 strains of Example 1 had hemolytic activity, the strains were plated on blood agar supplemented with 5% defibrinated sheep blood (MB cell, Korea) and cultured at 37°C for 24 hours, and then it was observed whether clear zones were formed around colonies.

As a result, as shown in FIG. 2, clear zones resulting from the lysis of blood cells were not observed around colonies, indicating that the CJRS-10671 and CJRS-10672 strains have no hemolytic activity.

### Example 4: Antibiotic resistance of strains

To determine antibiotic resistance of the CJRS-10671 and CJRS-10672 strains of Example 1, antibiotic resistance testing was conducted to determine the minimum inhibitory concentration (MIC) according to the EFSA guidelines for antibiotic resistance testing. The guidelines stipulate standards for cut-off values of eight types of antibiotics for *Leuconostoc mesenteroides.*

As a result of MIC testing, the CJRS-10671 and CJRS-10672 strains met the standards for five antibiotics, but exceeded the MIC standards for some antibiotics, and thus genetic analysis was conducted on whether the antibiotic resistance was intrinsic resistance or acquired resistance, according to the EFSA guidelines. The results are shown in Table 3 below. According to the EFSA guidelines, strains having intrinsic resistance can be used since there is no possibility of external transfer of an antibiotic resistant gene to other bacteria. Strains of the genus *Leuconostoc* have been reported to have resistance to chloramphenicol (MICs≥ 4 µg/mL), but it is known that such resistance is likely to be intrinsic resistance of the genus *Leuconostoc* and unlikely to result from horizontal gene transfer to other bacteria (AB Florez, 2005).

**TABLE 3**

| Antibiotics | *Leuconostoc mesenteroides* | | | |
|---|---|---|---|---|
| | Cut-off values (mg/L) | CJRS-10671 | CJRS-10672 | Susceptibility |
| Ampicillin | 4 | 0.5 | 0.5 | susceptible |
| Vancomycin | N.R. | N.R. | N.R. | N.R. |
| Gentamycin | 16 | 8 | 16 | susceptible |
| Kanamycin | 16 | 128 | 512 | resistance |
| Streptomycin | 34 | 128 | 128 | Moderate resistance |
| Erythromycin | 1 | 0.125 | 0.125 | susceptible |
| Clindamycin | 1 | 0.03125 | 0.0625 | susceptible |
| Tylosin | N.R. | N.R. | N.R. | N.R. |
| Tetracycline | 8 | 2 | 1 | susceptible |
| Chloramphenicol | 4 | 32 | 32 | Moderate resistance * Intrinsic resistance (AB Florez, 2005) |

### Example 5: Acid tolerance and bile salt tolerance of strains

Since stably reaching the intestine to exert effects is important in oral formulations, the strains were evaluated for acid tolerance and bile salt tolerance to determine suitability thereof as oral formulations. Specifically, acid tolerance testing was conducted wherein a strain subcultured for two passages was inoculated at 1% in a PBS solution adjusted to pH 3.0 by 1 N HCl and cultured at 37°C for 2 hours, and then a comparison was conducted between the initial viable cell count and the viable cell count after culturing.

The bile salt tolerance was determined wherein an activated strain was inoculated at 1% in a PBS solution containing 1% oxgall (Difco, USA) and cultured at 37°C for 2 hours, and then a comparison was conducted between the initial viable cell count and the viable cell count after culturing.

As a result, as shown in Table 4 below, the CJRS-10671 strain showed an acid tolerance of 95.7% and a bile salt tolerance of 136.6% based on 100% of the initial viable cell count, and the CJRS-10672 strain showed an acid tolerance of 120.6% and a bile salt tolerance of 67.9% based on 100% of the initial viable cell count.

**TABLE 4**

| Bacteria | Acid tolerance (%) | Bile salts tolerance (%) |
|---|---|---|
| CJRS-10671 | 95.7 ± 12.2 | 136.6 ± 24.6 |
| CJRS-10672 | 120.6 ± 31.1 | 67.9 ± 3.3 |

### Example 6: Intestinal adhesion of strains

To determine stability and suitability of the CJRS-10671 and CJRS-10672 strains of Example 1 in the intestinal environment, the strains were evaluated for intestinal adhesion by using HT-29 cell line (Korean Cell Line Bank, KCLB; Seoul, Korea) as intestinal epithelial cells. Specifically, the HT-29 cell line was cultured in Dulbecco's modified Eagle's minimal essential medium (DMEM) supplemented with 10% fetal bovine serum (FBS, Gibco, Carlsbad, CA, USA), 100 U/mL penicillin, and 0.1 mg/mL streptomycin under 37°C and a 5% CO₂ atmosphere. The HT-29 cell line was seeded at 1.0×10⁵ cells/24 wells so as to form about 80% of a monolayer, and then washed two times with PBS to remove the antibiotics contained in DMEM. Strains subcultured for two passages were washed with PBS and then re-dissolved in antibiotic-free DMEM, and used for treatment at a ratio of 2×10⁷ CFU/well. After culturing under 37°C and 5% CO₂ for 2 hours, non-adherent cells were removed by washing 3 times with PBS, and only the adherent HT-29 and strain cells were detached by addition of trypsin-ethylene-diamine-tetraacetic acid (EDTA), and then the viable cells were counted by using MRS agar. The degree of intestinal adhesion was expressed as the CFU number of the adherent strain cells per 100 HT-29 cells, and *Bifidobacterium bifidum*, *B. infantis*, and *B. lactis* ATCC type strains were used as controls.

As a result, it was confirmed that the intestinal adhesion of the CJRS-10671 strain was 217±21 bacteria/100 cells, which was equivalent to or higher than those of the control strains (FIG. 3).

### Example 7: Secretion of immune-enhancing cytokines by strains

### 7-1. Secretion of interleukin (IL)-6 in macrophage lines

It was determined whether the secretion of IL-6, known as an immune-enhancing cytokine, was induced by CJRS-10671 and CJRS-10672 of Example 1 in immune cells. A mouse macrophage cell line (Raw 264.7) and human monocytes (THP-1) were used as immune cells. The Raw 264.7 cell line was subcultured and maintained in DMEM containing 10% FBS and 1% Antibiotic-antimycotic one week before the experiment, and was seeded at 6×10⁴ cells/well in flat 96-well plates one day before the experiment. The THP-1 cell line was subcultured and maintained in RPMI medium containing 10% FBS, 1% penicillin-streptomycin (PS), and 0.05 mM 2-mercaptoethanol (2-ME), and seeded at 8×10⁴ cells/well in flat 96-well plates two days before the experiment, and then treated with 25 ng/mL phorbol 12-myristate 13-acetate (PMA) to induce the differentiation of human monocytes into human macrophages for two days. Thereafter, an antibiotic-free medium containing 10% FBS was used as a medium when the cells were co-cultured with CJRS-10671 and CJRS-10672. After culturing, CJRS-10671 and CJRS-10672 were measured and calculated for the total cell count by using a Novocyte instrument, and then used for treatment at a ratio of 1:10 with immune cells. After co-culturing for 24 hours, the cultured plates were centrifuged to settle cells and obtain only supernatants, which were then used to examine the cytokine secretion. Analytical samples were prepared using a BD Cytometric Bead Array (CBA) mouse/human flex set from BD Biosciences, and reading was conducted by a BD Lyric (flow cytometer), and the results were analyzed by FCAP software.

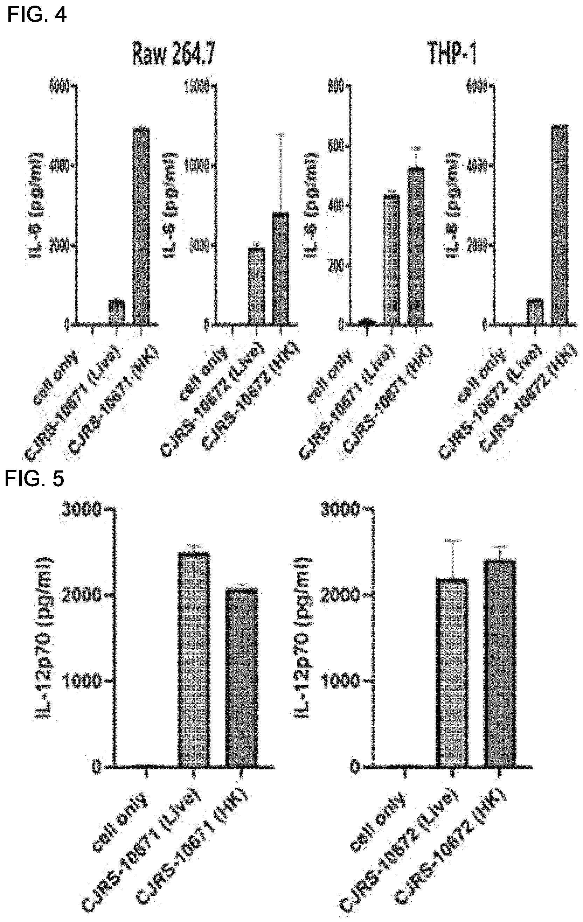

As a result, it was confirmed that CJRS-10671 and CJRS-10672 increased the secretion of IL-6 when co-cultured with mouse- or human-derived macrophages (FIG. 4).

### 7-2. Secretion of IL-12p70 in human peripheral blood mononuclear cells (PBMCs)

It was determined whether the secretion of IL-12p70, known as an immune-enhancing cytokine, was induced by CJRS-10671 and CJRS-10672 in human PBMCs. Human PBMCs were seeded at 2×10⁵ cells/well in 96-well plates. Thereafter, an antibiotic-free medium containing 10% FBS was used as a medium when the cells were co-cultured with CJRS-10671 and CJRS-10672. After culturing, CJRS-10671 and CJRS-10672 were measured and calculated for the total cell count by using a Novocyte instrument, and then used for treatment at a ratio of 1:10 with immune cells. Supernatants were obtained after culturing for 24 hours, and as a result of analysis, it was confirmed that CJRS-10671 and CJRS-10672 increased the secretion of IL-12p70 when co-cultured with human PBMCs (FIG. 5).

### 7-3. Secretion of interferon-gamma (IFNγ) of CD8 T cells in mouse splenocytes and human PBMCs

The IFNγ secretory capacity of CD8 T cells, cytotoxic T cells, is known to have a great effect on anticancer efficacy. To determine the IFNγ secretory capacity of cytotoxic T cells by CJRS-10671 and CJRS-10672 strains, CD8⁺ IFNγ⁺ was examined using mouse splenocytes and human PBMCs.

Specifically, for the isolation of mouse splenocytes, Hanks Balanced Salt Solution (HBSS) containing collagenase IV was inserted into the mouse spleen tissue by using a syringe to allow the cells to flow out. After repeating this procedure several times, the remaining tissue was cut into small pieces by using the syringe and incubated at 37°C for 25 minutes. After addition of 0.5 M EDTA, further incubation was conducted at 37°C for 5 minutes. The cells were stirred by a Pasteur pipette and then filtered through a cell strainer. After centrifugation at 1500 rpm for 3 minutes, red blood cells were removed by 1X RBC lysis buffer, and splenocytes were suspended in a fresh medium and used for the experiment.

Human PBMCs were purchased from STEMCELL Technologies and stored in a nitrogen tank after receipt before use in the experiment. On the day of the experiment, the stock was taken out of the nitrogen tank and melted in a constant-temperature water bath. RPMI 1640 supplemented with 10% FBS was added thereto, followed by centrifugation at 1500 rpm for 5 minutes, and then PBMCs were suspended in a fresh medium and used for the experiment.

A stimulator for T cells was required to determine the IFNγ secretory capacity in mouse splenocytes, and therefore, one day before co-culturing with the strains, 0.1 µg/mL anti-CD3 antibody diluted in PBS was coated at a concentration of 0.1 µg/mL with a volume of 100 µL per well in flat 96-well plates. On the day of co-culturing, the coated plates were prepared after washing two times with PBS, and the mouse splenocytes were isolated according to the protocol and then seeded at 5×10⁵ cells/well in 96-well plates coated while containing 1 µg/mL purified anti-CD28 antibody. The CJRS-10671 and CJRS-10672 strains were co-cultured with the splenocytes at a ratio of 1:10 using antibiotic-free culture media. After co-culturing for three days, brefeldin A was added, followed by Golgi-stop for 3-4 hours, and then markers to be checked using a BD transcription factor buffer set and fluorescent antibodies were stained to prepare analytical samples. Analytical samples were analyzed using a BD Lyric (flow cytometer) and Flowjo software, and then the results were evaluated.

Human PBMCs were seeded at 2×10⁵ cells/well, and then co-culturing was conducted by way of the same method as in Example 7-2.

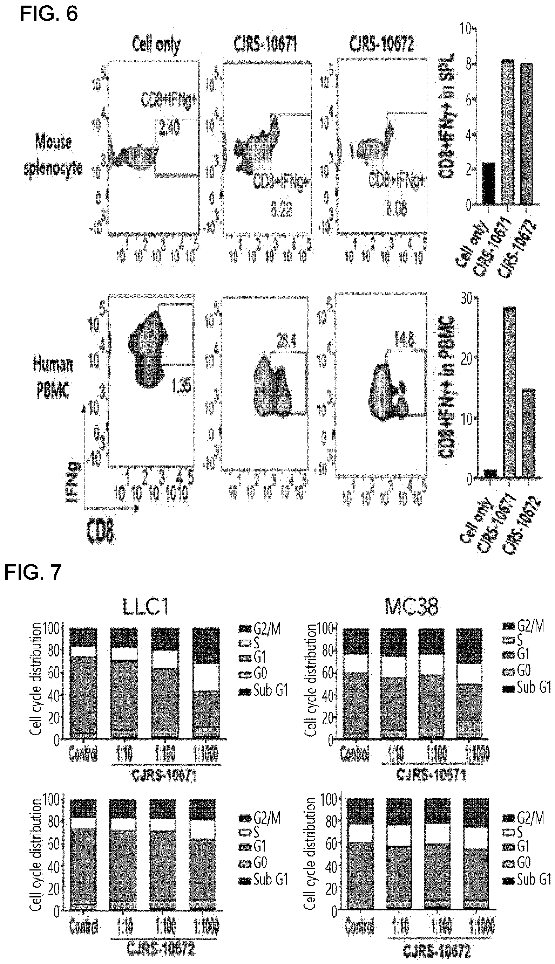

As a result, it was confirmed that CJRS-10671 and CJRS-10672 increased the IFNγ secretory capacity of CD8 T cells in mouse splenocytes and human PBMCs (FIG. 6).

### Example 8: Cell cycle regulation in cancer cells by strains

### 8-1. Cell cycle arrest in cancer cells

The cell cycle regulation in cancer cell lines by the CJRS-10671 and CJRS-10672 strains of Example 1 was examined. The lung cancer cell line Lewis lung carcinoma LL/2 (LLC1) and the colorectal cancer cell line murine carcinoma-38 (MC38) cells were used as cancer cell lines. One week before the experiment, the LLC1 and MC38 cell lines were cultured and maintained in DMEM containing 10% FBS and 1% Anti-anti. The LLC1 and MC38 cell lines were seeded at 2.4×10⁵ cells/well and 1.2×10⁵ cells/well in 6-well plates, respectively. To synchronize the cell cycle to the G0/G1 phase, the plates were washed two times with 1X PBS after 24 hours, and then the media were exchanged with antibiotic-free culture media containing 0.5% FBS. The CJRS-10671 and CJRS-10672 strains were added such that the ratio of cancer cell line : strain was 1:10, 1:100, or 1:1000. After co-culturing for 24 hours, the cells were detached using trypsin-EDTA and washed with 1X PBS, and then 5 mL of ice-cold 75% EtOH was added while only the cells were left, followed by storage at -20°C overnight. Thereafter, Ki67 and PI fluorescence staining was conducted, and the cell cycle results were analyzed using A BD Lyric (flow cytometer) and Flowjo software. The groups not treated with the CJRS-10671 and CJRS-10672 strains (strain-non-treatment groups) were used as control groups.

As a result, it was confirmed that the cell cycle arrest effect was observed in the strain treatment groups compared with the strain-non-treatment groups, and the cell cycle arrest effect was induced at the G2/M phase mainly by CJRS-10671 (FIG. 7).

### 8-2. Expression of cyclin B1 and cyclin dependent kinase 1 (CDK1), cancer cell cycle arrest-related factors

The changes in expression of cyclin B1 and CDK1, which are G2/M cell cycle regulation-related factors in the cancer cell line, by the CJRS-10671 and CJRS-10672 strains were examined. LLC1 cells were co-cultured with the CJRS-10671 and CJRS-10672 strains for 24 hours by way of the same method as in Example 8-1, and then RNA was extracted from the cells by using TRIzol. cDNA was synthesized therefrom, and PCR was performed using a QuantStudio 5 real-time PCR system to measure relative expression levels. The groups not treated with the CJRS-10671 and CJRS-10672 strains (strain-non-treatment groups) were used as control groups.

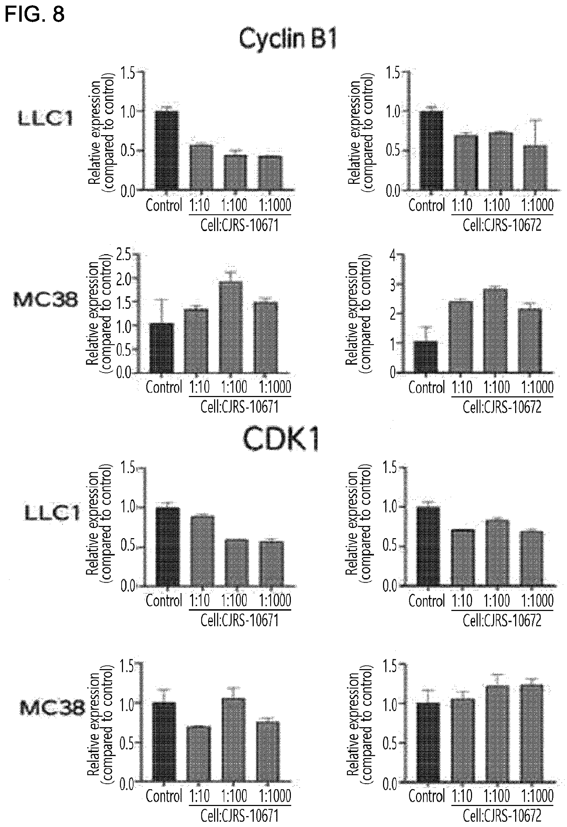

As a result, it was confirmed that cyclin B1 and CDK1 related with G2/M cell cycle arrest were reduced, and that the relative expression levels of cyclin B1 and CDK1 in LLC1 were mainly reduced in the groups treated with the strains compared with the strain-non-treatment groups (FIG. 8).

### Example 9: Cancer cell death by strains

To determine changes in survival rate of cancer cell lines by the CJRS-10671 and CJRS-10672 strains of Example 1, LLC1 and MC38 cells were seeded at 2.4×10⁵ cells/well and 1.2×10⁵ cells/well in 6-well plates, respectively. After 24 hours, the media were exchanged with media containing 0.5% FBS, followed by serum starvation for 24 hours. The CJRS-10671 and CJRS-10672 strains were used for treatment such that the ratio of cancer cell line : strain was 1:10, 1:100, or 1:1000. After co-culturing for 24 hours, the cells were detached using trypsin-EDTA and washed with 1X PBS, and fluorescent staining was conducted with Fixable Viability Stain 510 antibody to distinguish between dead cells and live cells, and the cell survival results were analyzed using BD Lyric (flow cytometer) and Flowjo software. Total cells were calculated as 100%, and the proportions of live cells and dead cells are shown in the drawing.

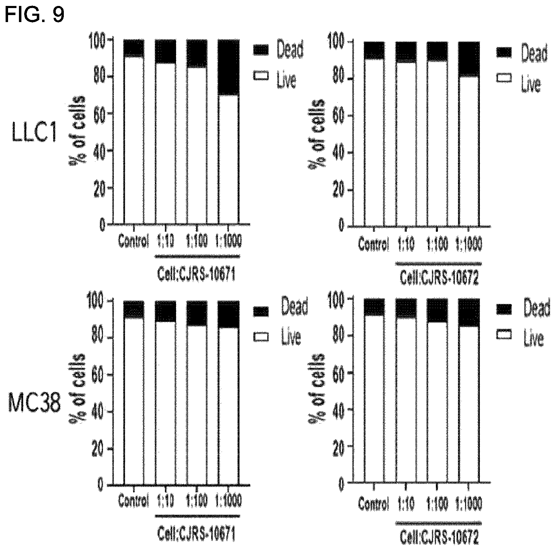

As a result, it was confirmed that the cell survival rate was reduced in the groups treated with the strains compared with the strain-non-treatment groups (FIG. 9).

### Example 10: Anticancer effect by strains in mouse lung cancer (LLC1) model

### 10-1. Tumor inhibitory effect

To construct a lung cancer animal model, the LLC1 cell line was subcutaneously injected at 4×10⁵ cells/mouse into c57BL/6 mice. When the tumor size reached about 30-50 mm³ after tumor cell injection, the mice were divided into the following groups: negative control group (non-treatment after tumor cell injection), CJRS-10671 or CJRS-10672 sample treatment group, immune checkpoint inhibitor anti-PD-1 treatment group (positive control group), and CJRS-10671 or CJRS-10672 sample and anti-PD-1 co-treatment group. The CJRS-10671 or CJRS-10672 sample was diluted in 100 µL of PBS to 10⁹ CFU per animal and administered a total of 10 times for 10 days, and anti-PD-1 (10 mg/kg, BioXCell) was intraperitoneally administered once every two days. The size of the tumor was measured by calipers three times a week, and on the next day after the end of administration, the mice were sacrificed to measure the tumor weight and analyze the blood and organs.

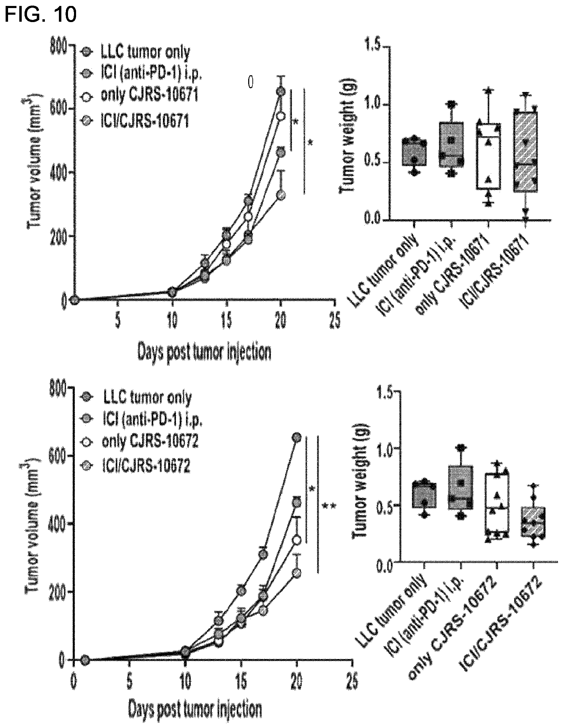

As a result, it was confirmed that CJRS-10671 reduced the tumor size even through the administration alone compared with the anti-PD-1 alone administration group and showed an excellent tumor inhibitory effect when co-administered with anti-PD-1. CJRS-10672 showed a slight effect through the administration alone, but showed a significantly excellent tumor-inhibitory effect when co-administered with anti-PD-1 (FIG. 10).

### 10-2. IFNγ secretory capacity of CD8 T cells

After the end of testing in Example 10-1, splenocytes were isolated from the spleen tissue of each group and then seeded at 10⁶ cells/well in round-bottom 96-well plates. The splenocytes were stimulated with PMA/lonomycin, which stimulates all immune cells, for 3-4 hours, and then subjected to surface marker staining and intracellular cytokine staining (ICS). Thereafter, the amount of IFNγ secreted by CD8 T cells was determined by FACS. To further determine the CD8 T cell potency, the same isolated splenocytes were stimulated with CD3/CD28 (2 µg/mL anti-CD3 antibody and 2 µg/mL anti-CD28 antibody), which stimulate T cells, for one day, and then subjected to surface marker staining and ICS. Thereafter, the amount of IFNγ secreted by CD8 T cells was determined by FACS.

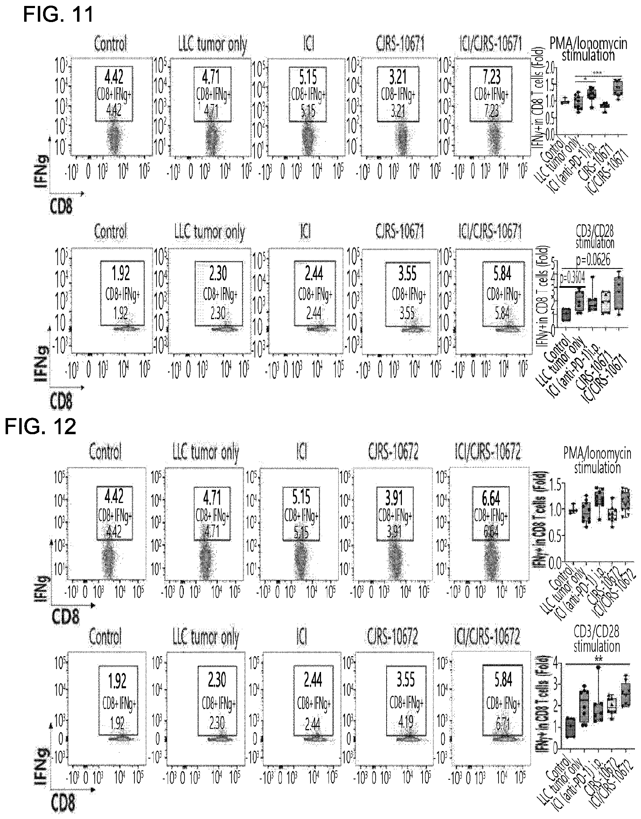

As a result, in the CJRS-10671 and anti-PD-1 co-administration group, it was confirmed that the amount of IFNγ secreted by CD8 T cells was significantly increased upon stimulation of all the immune cells and the amount of IFNγ secreted from CD8 T cells tended to increase even upon stimulation of T cells (FIG. 11). In the CJRS-10672 and anti-PD-1 co-administration group, the amount of IFNγ secreted by CD8 T cells was similar to that of the anti-PD-1 alone administration group upon stimulation of all the immune cells but the amount of IFNγ secreted from CD8 T cells was significantly increased upon stimulation of T cells (FIG. 12).

### 10-3. IFNγ secretory capacity of CD8 T cells in cancer tissue

After the end of testing of Example 10-1, tumor-infiltration lymphocytes (TILs) were isolated from the tumor tissue of each group, and then stimulated with CD3/CD28 (2 µg/mL anti-CD3 antibody and 2 µg/mL anti-CD28 antibody), which stimulate T cells, for one day, and then subjected to surface marker staining and ICS. Thereafter, the amount of IFNγ secreted by CD8 T cells was determined by FACS.

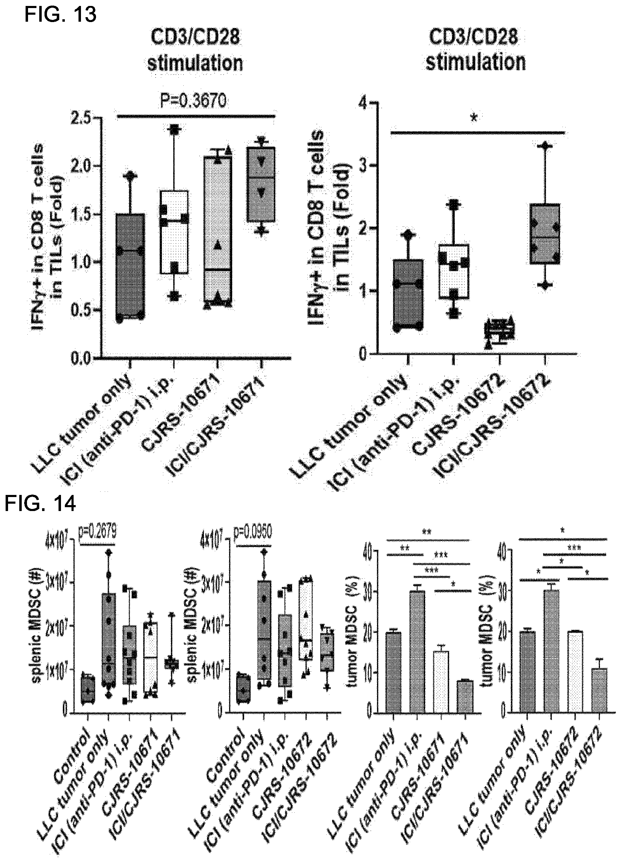

As a result, the amount of IFNγ secreted by CD8 T cells was increased upon simulation of T cells in both of the CJRS-10671 and anti-PD-1 co-administration group and the CJRS-10672 and anti-PD-1 co-administration group (FIG. 13).

### 10-4. Changes of immune suppressor cells

After the end of testing in Example 10-1, the changes of myeloid-derived suppressor cells (MDSCs) known as immune suppressor cells in the splenocytes and tumor cells of each group were determined by fluorescent staining using the CD11b⁺Gr-1⁺ marker and FACS.

As a result, MDSCs in the splenocytes were increased in the negative control group but was reduced in the CJRS-10671 and anti-PD-1 co-administration group and the CJRS-10672 and anti-PD-1 co-administration group. Also, it was confirmed that when the same marker was used in the tumor, MDSCs were significantly reduced in the CJRS-10671 and anti-PD-1 co-administration group and the CJRS-10672 and anti-PD-1 co-administration group, compared with the negative control group (FIG. 14).

### 10-5. Immune cell change profiling

After the end of testing in Example 10-1, splenocytes were isolated from the spleen tissue of each group, and then surface marker staining was conducted using fluorescent antibodies to determine changes of immune cells and immune suppressor cells. Fluorescent markers for respective types of immune cells were used as follows: M1 (CD11b⁺F4/80⁺CD86⁺), M2 (CD11b⁺F4/80⁺CD206⁺), DC (IA-IE⁺CD11b⁺CD11c⁺), B cell (CD3-B220⁺), NK cell (CD3⁻NK1.1⁺), NKT cell (CD3⁺NK1.1⁺), and Treg (CD4⁺CD25⁺Foxp3⁺). Analytical samples were analyzed using BD Lyric (flow cytometer) and Flowjo software, and then the results were evaluated as % and #.

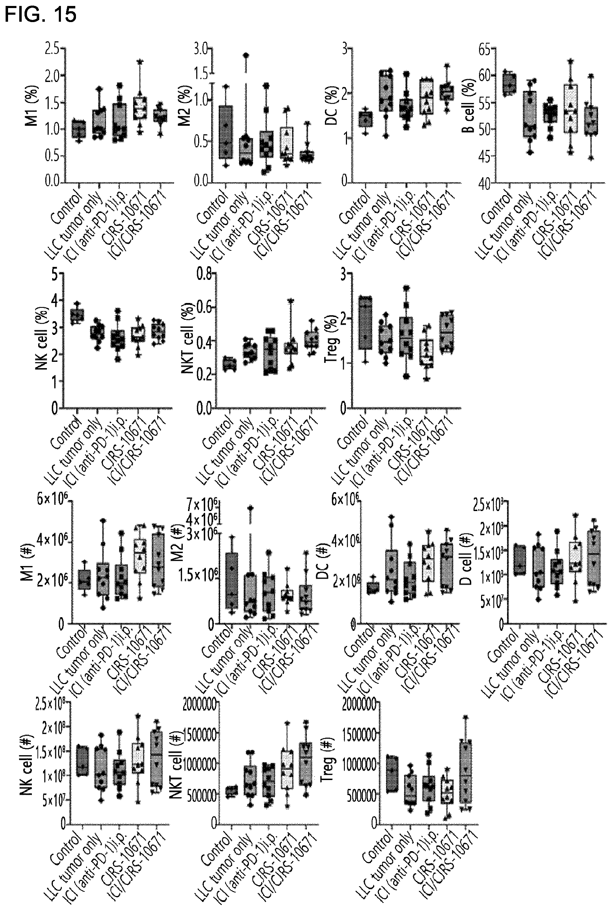

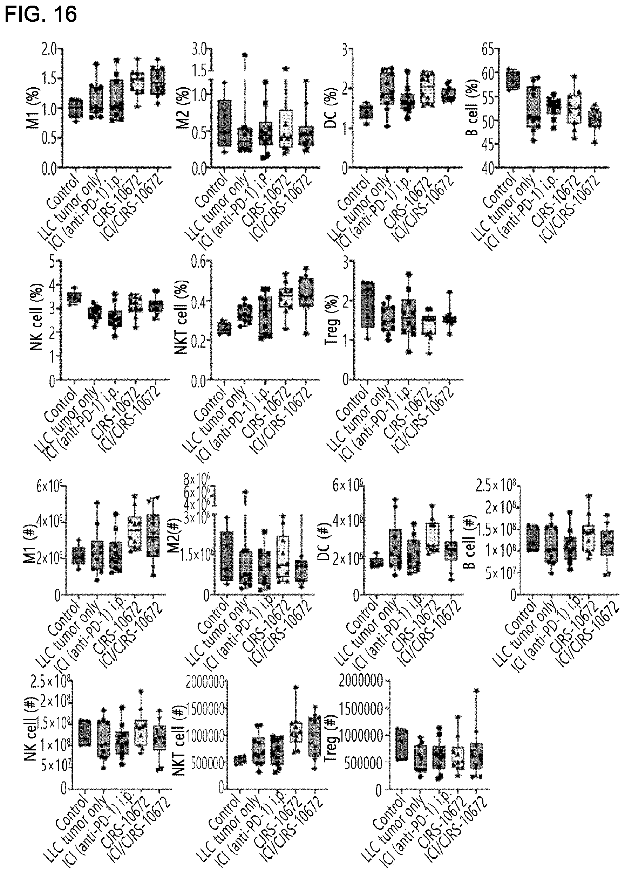

As a result, the % and # values of M1, DC, and NKT cells tended to increase in the groups of CJRS-10671 and CJRS-10672 alone or in combination with anti-PD-1 (FIGS. 15 and 16).

### Example 11: Anticancer effect by strains in mouse breast cancer (4T1) model

To construct a breast cancer animal model, a 4T1 cell line was subcutaneously injected at 3×10⁵ cells/mouse into BALB/c mice. When the tumor size reached about 30-50 mm³ after tumor cell injection, the mice were grouped into the following groups: negative control group (non-treatment after tumor cell injection), CJRS-10672 sample treatment group, immune checkpoint inhibitor anti-PD-1 treatment group (positive control group), and CJRS-10672 sample and anti-PD-1 co-treatment group. The CJRS-10672 sample was diluted in 100 µL of PBS to 10⁹ CFU per animal and administered a total of 10 times for 10 days, and anti-PD-1 (10 mg/kg, BioXCell) was intraperitoneally administered once every two days. The size of the tumor was measured by calipers three times a week, and on the next day after the end of administration, the mice were sacrificed to measure the tumor weight and analyze the blood and organs.

In addition, the number of the immune suppressor cell MDSCs in the obtained spleen-derived cells was determined by way of the same method as in Example 10-4.

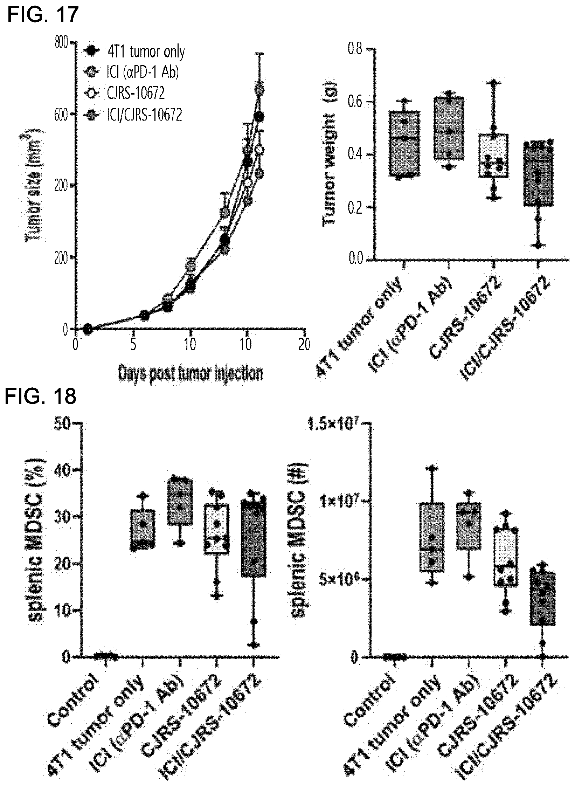

As a result, it was confirmed that CJRS-10672 reduced the size of breast cancer compared with the negative control group or the anti-PD-1 treatment group, and especially, individuals with reduced tumor weight and size were observed in the CJRS-10672 and anti-PD-1 co-administration group (FIG. 17).

The number of MDSCs as immune suppressor cells in the splenocytes was significantly reduced in the CJRS-10672 and anti-PD-1 co-administration groups (FIG. 18).

The CJRS-10671 and CJRS-10672 strains were deposited with the Korea Microorganism Conservation Center, a depository institution under the Budapest Treaty, on October 19, 2021, and given accession numbers KCCM13059P and KCCM13060P, respectively.

As set forth above, those skilled in the art to which the present disclosure pertains will be able to understand that the present disclosure may be embodied in other specific forms without departing from the technical spirit or essential characteristics thereof. Therefore, the embodiments described above should be construed as being exemplified and not limiting the present disclosure. It should be understood that all changes or modifications derived from the definitions and scopes of the claims and their equivalents fall within the scope of the disclosure.

## Claims

1. A pharmaceutical composition for preventing or treating lung cancer or breast cancer, the composition comprising, as an active ingredient, a *Leuconostoc mesenteroides* strain, a culture thereof, or a component derived therefrom.

2. A pharmaceutical composition for preventing or treating colorectal cancer, the composition comprising, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom, wherein the composition is administered in combination with an anticancer agent.

3. The composition of claim 1, wherein the *Leuconostoc mesenteroides* strain is at least one selected from *Leuconostoc mesenteroides* CJRS-10671 (accession number: KCCM13059P) and *Leuconostoc mesenteroides* CJRS-10672 (accession number: KCCM13060P).

4. The composition of claim 1, wherein the composition is administered in combination with an anticancer agent.

5. The composition of claim 2 or 4, wherein the anticancer agent is an anticancer immunotherapeutic agent.

6. The composition of claim 5, wherein the anti-cancer immunotherapeutic agent is at least one selected from the group consisting of anti-programmed cell death protein 1 (anti-PD-1), anti-programmed cell death-ligand 1 (anti-PD-L1), cytotoxic T lymphocyte associated antigen 4 (CTLA4), CTLA4/B7-1/B7-2 interaction inhibitors, cluster of differentiation 47/signal-regulatory protein (CD47/SIRP) interaction inhibitors, anti-T-cell immunoglobulin and mucin domain 3 (anti-Tim3), and anti-lymphocyte activating 3 (anti-LAG3).

7. The composition of claim 1 or 2, wherein the administration of the composition is performed in combination with anticancer therapy.

8. The composition of claim 7, wherein the anticancer therapy is at least one selected from the group consisting of radiotherapy, neoadjuvant therapy, chemotherapy, targeted therapy, and photodynamic therapy.

9. The composition of claim 1 or 2, wherein the composition is administered by at least one administration method selected from the group consisting of intraperitoneal administration, intravenous administration, intramuscular administration, subcutaneous administration, intradermal administration, oral administration, topical administration, intranasal administration, intrapulmonary administration, and intrarectal administration.

10. A food composition for preventing or alleviating lung cancer, breast cancer, or colorectal cancer, the composition comprising, as an active ingredient, a *Leuconostoc mesenteroides* strain, a culture thereof, or a component derived therefrom.

11. A health functional food for preventing or alleviating lung cancer, breast cancer, or colorectal cancer, the health functional food comprising, as an active ingredient, a *Leuconostoc mesenteroides* strain, a culture thereof, or a component derived therefrom.

12. A feed composition for preventing or alleviating lung cancer, breast cancer, or colorectal cancer, the composition comprising, as an active ingredient, a *Leuconostoc mesenteroides* strain, a culture thereof, or a component derived therefrom.

13. A food composition for preventing or alleviating cancer or inflammation, the composition comprising, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

14. A health functional food for preventing or alleviating cancer or inflammation, the health functional food comprising, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

15. A feed composition for preventing or alleviating cancer or inflammation, the composition comprising, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

16. A food composition for immune enhancement, the composition comprising, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

17. A health functional food for immune enhancement, the health functional food comprising, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.

18. A feed composition for immune enhancement, the composition comprising, as an active ingredient, *Leuconostoc mesenteroides* CJRS-10671 (accession number: KCCM13059P) or *Leuconostoc mesenteroides* CJRS-10672 (accession number: KCCM13060P); a culture thereof; or a component derived therefrom.
